# FASCICULE DE BREVET EUROPEEN

(11) **EP 2 429 499 B1**
(45) Date de publication et mention de la délivrance du brevet: **25.03.2015**
(21) Numéro de dépôt: 10721462.9
(22) Date de dépôt: 12.05.2010
(51) Int. Cl.: A61K 9/18, A61K 31/165, A61K 31/192, A61K 31/216, A61K 31/475, A61P 25/18, A61P 13/10, A61P 21/04, A61P 29/00

(54) **PROCEDE D'IMPREGNATION PAR CO2 SUPERCRITIQUE**
IMPRÄGNIERUNGSVERFAHREN MIT ÜBERKRITISCHEM CO2
METHOD FOR IMPREGNATION WITH SUPERCRTITICAL CO2

(30) Priorité: 15.05.2009 FR 0953221
(43) Date de publication de la demande: 21.03.2012
(73) Titulaire: Pierre Fabre Médicament, 92100 Boulogne-Billancourt (FR)
(72) Inventeur: LOCHARD, Hubert, F-81600 Brens (FR); FREISS, Bernard, F-81100 Castres (FR)
(74) Mandataire: Regimbeau
(86) Numéro de dépôt international: PCT/EP2010/056582
(87) Numéro de publication internationale: WO 2010/130799

(56) Documents cités:
- EP-A- 1 894 565
- WO-A-94/18264
- WO-A-99/25322
- CORTESI A ET AL: "Supercritical fluids chromatography for impregnation optimization", JOURNAL OF SUPERCRITICAL FLUIDS, PRA PRESS, US, vol. 19, no. 1, 15 novembre 2000 (2000-11-15), pages 61-68, XP004262617, ISSN: 0896-8446

## Description

La présente invention concerne un procédé d'imprégnation d'un excipient pharmaceutique polymérique par une substance active à l'aide de CO₂ supercritique.

Des procédés d'imprégnation utilisant le CO2 supercritique ont déjà été décrits dans l'art antérieur. Toutefois aucun d'entre eux ne décrit un procédé rapide, facile à mettre en oeuvre, utilisable quelque soit le type d'excipient polymérique et en particulier un excipient polymérique plutôt connu en tant que désintégrant qu'en tant que liant, et utilisable avec tout type de substance active, qu'elle soit soluble ou non en milieux aqueux.

Ainsi l'article de Manna, et al (The Journal of Supercritical Fluids, Volume 42, Issue 3, October 2007, Pages 378-384) décrit un procédé continu d'imprégnation de polyvinylpyrrolidone (PVP) par du kétoprofène. Toutefois le PVP utilisé est non réticulé. Or le PVP non réticulé est un agent liant bien connu pour obtenir des formes amorphes stabilisées, quel que soit le procédé d'imprégnation utilisé. En outre le procédé décrit est très lent car lié à la solubilité de l'actif dans le CO₂ (un essai = 5 jours). Il n'est donc pas utilisable avec des substances actives insolubles dans le CO₂ supercritique contrairement au procédé selon la présente invention. En outre dans ce procédé il n'y a pas de mélange préalable entre le kétoprofène et le PVP puisqu'au contraire le mélange préalable est celui entre la substance active et le CO₂ supercritique. L'article d'Ugaonkar et al (International. Needham Journal of Pharmaceutics, Volume 333, Issues 1-2, 21 March 2007, Pages 152-161) décrit l'effet du n-scCO₂ sur la conversion du carbamazepine de sa forme cristalline à sa forme amorphe. Toutefois, le procédé décrit n'utilise pas du CO₂ supercritique mais du CO₂ liquide (P= 63 bars, T= 25°C) appelé near supercritical CO₂. En outre avant chaque essai; les auteurs réalisent un «pré-séchage» du polymère à 80°C pendant 16 h ce qui rallonge le procédé. D'après les résultats par DRX indiqués dans le tableau 1, une amorphisation significative mais partielle n'est obtenue qu'avec le PVP à faible masse moléculaire, c'est-à-dire le PVP non réticulé. Ces résultats sont confirmés par DSC ou l'atténuation est très limitée. La très forte variabilité analytique qu'on observe dans les résultats décrits dans cet article s'explique par des différences de granulométrie très importantes entre les grosses particules de polymère et le principe actif. Les différences de dissolution observées sont insignifiantes et imputables à la présence de l'excipient (pas de différence entre treated vs. Untreated). Les auteurs ne prouvent pas que les formes partiellement amorphes soient stabilisées. Les auteurs concluent que c'est l'interaction CO₂ / polymère qui permet l'amorphisation (page 160) et que l'importance de la flexibilité des chaînes de polymère est majeure. Ainsi donc les auteurs indiquent que seuls les polymères présentant une bonne flexibilité de chaîne (tel que le PVP non réticulé) permettent d'obtenir l'amorphisation de la substance active lors de son imprégnation dans l'excipient. Ainsi donc selon les auteurs il n'est pas possible d'utiliser le procédé décrit dans cet article avec n'importe quel excipient et en particulier avec des polymères dont les chaînes sont assez rigides tels que le PVP réticulé.

L'article de Banchero et al (The Journal of Supercritical Fluids, In Press, Corrected Proof, Available online 30 January 2009) décrit un procédé d'imprégnation du PVP par le piroxicam en utilisant un solvant supercritique. Toutefois, le procédé décrit est particulièrement long : entre 20-48h. En outre la pression utilisée est très élevée : environ 300 bars. Il en est de même de la température de travail : environ 100°C. Enfin le PVP utilisé est du PVP non réticulé, un agent liant bien connu pour obtenir des formes amorphes stabilisées de principe actif, quel que soit le procédé d'imprégnation utilisé.

L'article d'Albertini et al (European journal of pharmaceutics and biopharmaceutics 56 (2003) p 479-487) décrit un procédé d'amorphisation du piroxicam par une simple granulation à la vapeur d'eau (60°C, sous vide) en utilisant comme excipient du b-lactose, du PVP K12 et du PVP K90. Cela conduit à une amorphisation stabilisée sur 1 an (d'ailleurs p 387, les auteurs précisent que le PVP non réticulé est bien connu en tant qu'inhibiteur de cristallisation des substances actives).

L'article de Gong et al (Journal of Pharmaceutical and Biomédical Analysis, Volume 48, Issue 4, 1 December 2008, Pages 1112-1119) décrit un procédé d'imprégnation d'hydroxypropylméthylcellulose par de l'indométacine en utilisant du CO₂ supercritique. Toutefois le procédé est mis en oeuvre avec agitation lors de l'étape utilisant le CO₂ supercritique (180 tours/minutes) : cela rend le procédé non-viable économiquement en raison du surcoût très important car il y a nécessité d'un système d'agitation sous pression. En outre l'autoclave ne doit être rempli que très partiellement pour permettre le brassage du CO₂ supercritique autour de la poudre (le coût financier du brassage directe de poudre en enceinte pressurisée est prohibitif). Par ailleurs avec le procédé décrit, seul les essais à température très élevés: 110 °C et 130°C ont permis d'obtenir une amorphisation (ces températures sont très proches de la température de fusion du principe actif (160 °C)).

Les inventeurs ont découvert de façon surprenante un nouveau procédé simple et facile, n'ayant pas les inconvénients de l'art antérieur, et en particulier utilisable quelque soit le type d'excipient pharmaceutique polymérique, en particulier avec des excipient qui ne sont pas connus en tant qu'inhibiteur de cristallisation des substances actives, et quel que soit le type de substances actives, par exemple avec des substances actives solubles en milieu aqueux, permettant d'obtenir l'imprégnation d'un excipient polymérique pharmaceutique par une substance active, la substance active se trouvant ainsi sous une forme amorphe stabilisée. Un tel procédé comprend une étape de diffusion moléculaire en mode statique à l'aide de CO₂ supercritique.

Des procédés utilisant une telle étape ont déjà été décrits dans l'art antérieur. Toutefois ils n'ont jamais été utilisés sans agitation pour l'imprégnation d'un excipient pharmaceutique polymérique selon l'invention, et en particulier d'un excipient non poreux, sans ajout de solvant autre que le CO₂ supercritique.

Ainsi, la demande de brevet WO03/043604 décrit un procédé d'inclusion d'une substance active dans un support poreux comprenant une étape de diffusion moléculaire en mode statique. Toutefois un tel procédé permet l'obtention d'un complexe (ou composé d'inclusion) entre la substance active et le support poreux et non l'obtention d'un excipient polymérique imprégné par la substance active. En outre le support utilisé est poreux et n'est pas un excipient polymérique puisqu'il s'agit de cyclodextrine. De plus le procédé n'est utilisé qu'avec des substances actives bien précises, c'est à dire une substance active peu soluble dans un milieu aqueux, le support poreux étant quant à lui soluble. En outre le procédé est assez long puisque l'étape de diffusion moléculaire dure au minimum 16 heures. Enfin ce procédé comprend obligatoirement une étape de lavage par du CO₂ supercritique du complexe obtenu, ce qui rallonge d'autant le procédé.

La demande de brevet WO2004/096284 décrit un procédé de préparation de complexes moléculaires solubles comprenant une étape de diffusion moléculaire en mode statique. Toutefois un tel procédé permet l'obtention d'un complexe entre la substance active et la molécule hôte et non l'obtention d'un excipient polymérique imprégné par la substance active. En effet la complexation est l'inclusion d'une molécule invitée (ici la substance active) dans la cavité d'une molécule hôte. Elle est déterminée par un équilibre de complexation définit par une constante Ks. La molécule hôte présente donc obligatoirement une cavité et est donc poreuse, ce qui n'est pas le cas de l'excipient pharmaceutique polymérique selon l'invention. En outre la molécule hôte n'est pas un excipient polymérique puisqu'il s'agit de cyclodextrine. En outre le procédé comprend obligatoirement la présence d'un autre solvant que le CO₂ supercritique appelé agent de diffusion tel que par exemple de l'eau. Comme le montrent les exemples, l'absence de cet agent empêche la formation de complexes moléculaires. Il ne permet donc pas l'obtention d'une poudre sèche directement après l'étape de diffusion moléculaire contrairement au procédé selon l'invention. De plus le procédé n'est utilisé qu'avec des substances actives bien précises, c'est à dire une substance active peu soluble dans un milieu aqueux, la molécule hôte étant quant à elle soluble.

Le brevet US 6,414,050 décrit quant à lui un procédé de préparation d'une composition par mise en contact à l'aide de CO₂ supercritique d'un substrat de polymère et d'un substrat de matériau biofonctionnel. Toutefois, les conditions de pression et de température choisies pour cette mise en contact sont telles qu'elles permettent de réduire la viscosité du polymère pour le plastifier (c'est à dire qu'il y a ramollissement) ou le fondre et/ou le gonfler. Les conditions sont donc assez drastiques et il y a une modification visible à l'oeil nu de la structure du polymère. En outre la composition obtenue est poreuse puisque en raison de la liquéfaction du polymère, lorsque le CO₂ est éliminé il forme de bulles dans le polymère qui est en train de se solidifier. De plus dans tous les exemples, l'étape de mise en contact a été mise en oeuvre avec agitation, ce qui pose des problèmes d'industrialisation du procédé en raison des surcoûts que cela entraîne. En outre il n'y a pas de modification de la forme physique de la substance active, c'est-à-dire qu'il n'y a pas d'amorphisation de cette substance active par la procédé décrit. De plus, il n'y a pas de mélange préalable entre la substance active et le polymère puisque que l'étape de mise en contact avec du CO₂ supercritique a lieu sous agitation. Enfin, pour obtenir une poudre, il est nécessaire de pulvériser le produit obtenu à travers une buse de pulvérisation ce qui complexifie le procédé.

La demande de brevet WO 94/18264 décrit l'imprégnation d'un polymère avec un principe actif en utilisant le CO₂ supercritique. Toutefois, pour réaliser cette imprégnation, un liquide est ajouté au mélange, ce liquide pouvant en particulier être de l'eau et est en général un solubilisant de la substance active. En outre, dans les exemples le principe actif est en solution (ex 24-26, page 46, 127), et non à l'état solide. De plus, dans les exemples 2 et 4 du tableau réalisés sans présence d'eau, et donc sans ajout de liquide, et soit dans deux fioles séparées, soit dans la même fiole, à 60 degrés et 13,8 MPa, il n'y a pas d'imprégnation des billes qui restent blanches.

Donc le procédé semble ne pas pouvoir être mis en oeuvre, selon ce document, en absence de liquide et en particulier en absence d'eau.

Or, la présence d'un liquide a pour désavantage l'obligation de devoir l'éliminer à la fin du procédé d'imprégnation.

La demande WO 99/25322 divulgue l'imprégnation de la polyvinyl pyrrolidone réticulée ou de l'amidon glycolate natrium réticulé avec des principes actifs comme le kétoprofène en utilisant le CO₂ supercritique par un procédé continu.

Le principe actif est tout d'abord solubilisé dans le fluide supercritique avant son ajout au polymère. Le problème de ce type de procédé est qu'il est uniquement utilisable pour des substances actives solubles dans le CO₂ supercritique.

En outre, bien que le mode statique soit divulgué dans le document D2, seul le mode dynamique est présent dans les exemples.

En partant des données décrites dans cette demande on peut facilement accéder à la quantité de CO₂ nécessaire pour traiter 1 kg. Dans l'exemple 1, l'auteur imprègne 1,225 g de Nimesulide dans 5g de PVP réticulé. Il utilise pour cela 43 kg de CO₂ (pour une densité du CO₂ liquide = 0,9 kg / litre environ), soit 35 tonnes de CO₂ /Kg de Principe actif

Dans l'exemple 2, l'auteur imprègne 1,06 g d'aciclovir dans 5g de Méthacrylate réticulé. Il utilise pour cela 130 kg de CO₂ (pour une densité du CO₂ liquide = 0,9 kg / litre environ), soit 122 tonnes de CO₂ /Kg de Principe actif

Selon l'article de A. Bounaceur et al. / (J. of Supercritical Fluids 41 (2007) 429-439), la fraction molaire du Kétoprofène dans le CO₂ pure est d'environ y = 4.1 × 10-5 (200 bars, 65°C). Donc, pour imprégner du Kétoprofène selon ce document, il faut utiliser au minimum 4,3 tonnes de CO₂ /Kg de Principe actif.

La teneur en CO₂ à utiliser est donc trop importante pour pouvoir industrialiser ce procédé qui a donc un coût en CO₂ prohibitif.

Les inventeurs ont donc découvert un procédé alliant les avantages de l'utilisation d'une étape de diffusion moléculaire en mode statique avec du CO₂ supercritique, sans les désavantages de l'art antérieur, tels que par exemple :
- la nécessité d'utiliser un excipient poreux ou
- un procédé trop long par exemple en raison d'un nombre d'étape trop important (lavage supplémentaire avec CO₂ supercritique ou pulvérisation avec une buse), ou
- des conditions de température et de pression trop drastiques ce qui provoque la liquéfaction ou au moins le ramollissement du polymère ou ce qui rallonge le procédé ou
- l'utilisation de l'agitation lors de l'étape de diffusion moléculaire,
- l'utilisation d'une quantité trop importante de CO₂ ou
- la présence obligatoire d'un autre fluide en plus du CO₂.

La présente invention concerne donc un procédé batch d'imprégnation d'un excipient pharmaceutique polymérique par une substance active, caractérisé en ce qu'il comprend les étapes successives suivantes :
a) mélange de la substance active et de l'excipient pharmaceutique polymérique non poreux, l'excipient pharmaceutique polymérique étant sous forme solide et insoluble dans le CO₂ supercritique et n'étant pas de la polyvinylpyrrolidone non réticulé;
b) mise en oeuvre d'une étape de diffusion moléculaire en l'absence d'eau par mise en contact en mode statique sans agitation du mélange obtenu à l'étape a) avec du CO₂ supercritique à une pression comprise entre 80 et 170 bars et à une température comprise entre 31 et 90°C pendant entre 1 et 6 heures,
c) récupération de l'excipient pharmaceutique polymérique imprégné par la substance active obtenu à l'étape b), l'excipient pharmaceutique polymérique imprégné n'étant pas poreux et étant sous forme solide et la substance active étant sous forme amorphe,
le procédé étant mis en oeuvre en l'absence de solvent additionnel.

En utilisant le procédé selon la présente invention, la quantité de CO₂ nécessaire pour traiter 1 kg de PA est uniquement fonction de la densité apparente de la poudre et de la teneur en actif imprégné.

Ainsi, en émettant les hypothèses suivantes:
Densité apparente de poudre = 500 g/litre
Teneur en actif imprégné = 20 %

Il faut utiliser uniquement 10 kg de CO₂ /Kg de Principe actif.

Ainsi, ce procédé peut être mis en oeuvre en utilisant 400 fois moins de CO₂ pour le kétoprofène et 12000 fois moins de CO₂ pour l'aciclovir que dans le procédé décrit dans la demande de brevet WO 99/25322. Cela réduit d'autant le cout « solvant » et réduit énormément les investissements nécessaires.

Dans le cadre de la présente invention, l'excipient pharmaceutique polymérique utilisé est non poreux. Il ne s'agit donc pas d'une cyclodextrine ou d'une molécule hôte ayant la forme d'une cage et permettant la formation d'un complexe moléculaire. En effet le produit obtenu par le procédé selon la présente invention n'est pas un complexe entre l'excipient et la substance active mais un excipient imprégné par la substance active. Le produit obtenu est donc une dispersion solide au niveau moléculaire entre l'excipient pharmaceutique polymérique et la substance active et non une insertion de la substance active dans la cavité d'un excipient poreux. Ainsi la substance active imprégnant l'excipient polymérique se trouve sous forme amorphe. Il y a donc eu en particulier modification physique de la substance active lors de son imprégnation.

Au sens de la présente invention on entend par « excipient polymérique » tout excipient polymérique ayant une longue chaîne. Il ne s'agit donc pas d'un oligomère tel qu'une cyclodextrine ou le lactose. En particulier la chaîne polymérique de l'excipient polymérique selon l'invention comprend au moins 10 motifs par exemple au moins 20 motifs.

Au sens de la présente invention, on entend par « excipient pharmaceutique » tout excipient utilisable en milieu pharmaceutique, alimentaire ou vétérinaire. En particulier il peut s'agir d'un excipient ayant la fonction d'agent diluant, liant, enrobant, anti-adhérent, désintégrant, fluidifiant, solubilisant, lubrifiant, stabilisant, antiagglomérant, anti-humidité, de masquage de gout ou de charge, de modification du profil de libération (à libération prolongée par exemple) etc.

Dans un mode de réalisation particulier, il s'agit d'un polymère cellulosique, tel que par exemple cellulose, cellulose microcrystalline, hypromelose, en particulier d'acétate succinate, méthylcellulose, éthylcellulose, hydoxypropylcellulose, hydroxypropylméthylcellulose ou carboxyméthylcellulose, d'une cire, d'une gomme végétale ou de synthèse, telle que par exemple gomme de guar, d'acacia, de xanthane ou de caroube, d'un polyéthylène glycol, d'un polymère phtalique tel que par exemple la cellulose acéto-phtalate, de la polyvinylpyrrolidone réticulé, de l'amidon ou une maltodextrine ou un mélange de ceux-ci. En particulier, il ne s'agit pas d'un polysaccharide et/ou d'un polyose. Dans un autre mode de réalisation particulier, l'excipient pharmaceutique polymérique est choisi dans le groupe constitué par un polymère cellulosique, la polyvinylpyrrolidone réticulé et leur mélange, en particulier dans le groupe constitué par la carboxyméthylcellulose par exemple de sodium, l'hydroxypropylméthylcellulose, la méthylcellulose, la polyvinylpyrrolidone réticulé et leur mélange.

L'excipient pharmaceutique polymérique selon l'invention se trouve sous forme solide à température ambiante et donc lors de l'étape a) du procédé selon l'invention. En outre sa température de transition vitreuse (Tg) et de fusion (Tf) est telle que lors de l'étape b) l'excipient polymérique reste sous forme solide et donc ne se liquéfie pas. Il n'y a donc pas gonflement et plastification de l'excipient polymérique lors de cette étape. Il y a cependant une augmentation de la mobilité des chaînes polymérique lors de cette étape b), ce qui permet la pénétration de la substance active dans l'excipient polymérique et donc son imprégnation par la substance active.

L'excipient polymérique selon l'invention n'est pas de la polyvinylpyrrolidone non réticulée. En effet un tel excipient est déjà bien connu comme inhibiteur de cristallisation des substances actives et n'a donc pas besoin d'être imprégné par le procédé selon la présente invention. En outre la mobilité de ses chaînes polymériques est particulièrement importante. Le procédé selon la présente invention s'adresse plutôt à des excipients pharmaceutiques polymériques dont les chaînes polymériques sont peu mobiles en particulier à température ambiante tel que le PVP réticulé.

L'excipient pharmaceutique polymérique utilisable dans le procédé selon la présente invention est en particulier insoluble en milieu aqueux.

De façon surprenante, les inventeurs se sont aperçus que le procédé selon la présente invention permet de surexprimer les fonctionnalités de l'excipient pharmaceutique polymérique utilisé. En effet si un excipient est par exemple un agent désintégrant (comme par exemple la polyvinylpyrrolidone réticulée ou la carboxyméthylcellulose de sodium), suite à son imprégnation par la substance active à l'aide du procédé selon la présente invention son pouvoir désintégrant est considérablement amélioré (c'est-à-dire presque doublé). Par ailleurs il est tout à fait étonnant de pouvoir imprégner un excipient polymérique pharmaceutique ayant la fonction d'agent désintégrant par une substance active et d'obtenir une forme amorphe stabilisée de la substance active.

Par « substance active » on entend au sens de la présente invention, toute substance active qu'elle soit soluble ou peu soluble dans un milieu aqueux. Ce procédé n'est donc pas limité aux substances actives peu solubles dans un milieu aqueux. Le but de l'imprégnation n'est donc pas uniquement d'améliorer la dissolution de la substance active en milieu aqueux, mais principalement de permettre d'obtenir une forme amorphe stabilisée de la substance active. Ainsi, en particulier on utilisera une substance active dont la forme amorphe n'est pas stabilisée. Dans un mode de réalisation particulier lors de l'étape a) la substance active se trouve sous forme cristalline. En outre lors de l'étape c) la substance active est sous forme amorphe. Il y a donc eu amorphisation de la substance active lors de l'étape b), c'est-à-dire lors de son imprégnation de l'excipient pharmaceutique polymérique. La substance active peut-être un actif pharmaceutique (on peut citer à titre d'exemple les analgésiques, les antipyrétiques, l'aspirine et ses dérivés, les antibiotiques, les anti-inflammatoires, les antiulcéreux, les antihypertenseurs, les neuroleptiques, les antidépresseurs, les oligonucléotides présentant une activité thérapeutique, les peptides présentant une activité thérapeutique et les protéines présentant une activité thérapeutique), cosmétique ou nutraceutique ou un mélange de celles-ci. En particulier, la substance active est insoluble dans le CO₂ supercritique (telle que par exemple la vinflunine).

Par « substance active peu soluble dans un milieu aqueux », on entend au sens de la présente invention toute substance active peu ou pas soluble dans un milieu aqueux et ayant en particulier une solubilité inférieure à au moins 20 µg/ml.

En particulier, la substance active selon l'invention est choisie dans le groupe constitué par les dérivés d'anilide, les dérivés d'épipodophyllotoxine, le minoxidil, le piroxicam, l'acide valérique, l'acide octanoïque, l'acide laurique, l'acide stéarique, l'acide tiaprofénique, l'oméprazole, l'éconazole, le miconazole, le kétoconazole, l'astémizole, la cyclobenzaprine, la nimésulide, l'ibuprofène, la téfénadine, le dompéridone, le naproxen, l'éflucimibe, le kétoprofène, la vinflunine, le milnacipran, le fénofibrate, le sulfate ferreux monohydrate, le sulfate ferreux heptahydrate et leur mélange, plus particulièrement choisie dans le groupe constitué par le kétoprofène, la vinflunine, le milnacipran, le fénofibrate, le sulfate ferreux monohydrate, le sulfate ferreux heptahydrate et leur mélange.

Le procédé selon la présente invention est particulièrement intéressant pour l'imprégnation :
- de kétoprofène dans l'excipient choisi parmi : méthylcellulose, PVP réticulé, HPMC et carboxyméthycellulose,
- de vinflunine dans la carboxyméthyl cellulose,
- de minalcipran dans l'HPMC ou la méthylcellulose

Par « CO₂ supercritique », on entend au sens de la présente invention le CO₂ utilisé à une température et une pression supérieure à sa valeur critique.

Par « mode statique » on entend au sens de la présente invention une réaction ou un procédé dans lequel tous les réactifs sont mis simultanément en présence et où on laisse la réaction se dérouler. Par exemple, dans l'étape b) de la présente invention, on met dans un autoclave le mélange obtenu à l'étape a) et du CO₂ supercritique et on laisse réagir pendant plusieurs heures. La masse de produit n'évolue pas durant la réaction. A l'inverse, en mode dynamique, les réactifs sont apportés au fur et à mesure de l'évolution de la réaction ou de la production. Souvent dans le cadre d'un mode dynamique, il y a circulation d'un fluide ou agitation. La masse de produit évolue durant la production.

En outre le procédé selon l'invention est un procédé batch. Ainsi tous les ingrédients nécessaires à l'imprégnation sont apportés en une seule fois au début de l'étape b) et l'excipient polymérique imprégné est obtenu à la fin de l'étape b).

Aucun autre ingrédient n'est ajouté ou enlevé entre le début et la fin de l'étape b).

Dans un mode de réalisation particulier, le ratio massique entre le principe actif et l'excipient pharmaceutique polymérique dans l'excipient pharmaceutique polymérique imprégné est compris entre 1 et 60%, en particulier entre 1 et 50%, plus particulièrement entre 20 et 35%, par exemple entre 10 et 35%.

L'étape a) du procédé selon la présente invention est très importante car elle permet le mélange intime des deux ingrédients (excipient pharmaceutique polymérique + substance active) avant l'étape de diffusion moléculaire, qui elle est mise en oeuvre sans agitation. Par « mélange intime » de A et de B on entend un mélange de A et de B dans lequel A et B se retrouvent uniformément répartis au sein du mélange obtenu. L'étape a) permet donc de réduire la durée de l'étape b). En particulier cette étape est mise en oeuvre avec un mélangeur du type mélangeur à tambour, mélangeur convectif, mélangeur fluidisé ou mélangeur statique.

Particulièrement la substance active lors de l'étape a) est sous forme solide, plus particulièrement sous forme de poudre, par exemple sous forme cristalline. Dans un mode de réalisation particulier lors de l'étape a), la substance active et l'excipient pharmaceutique polymérique sont sous forme solide, en particulier sous forme de poudre. Le mélange obtenu à la fin de l'étape a) est donc par exemple un mélange physique, de façon particulière une poudre sèche.

Particulièrement l'étape a) est mise en oeuvre à température et pression ambiante. L'étape b) du procédé selon la présente invention permet l'imprégnation de l'excipient pharmaceutique polymérique. L'utilisation du CO₂ supercritique évite l'utilisation d'un solvant organique qui devra être ensuite éliminé de l'excipient pharmaceutique polymérique imprégné obtenu.

Dans un mode de réalisation particulier, l'étape b) est mise en oeuvre dans un réacteur fermé, en particulier un autoclave. Le mélange obtenu à l'étape a) est donc introduit dans ce réacteur, de façon concomitante ou successive avec le CO₂. Le CO₂ est introduit sous forme de gaz. Le réacteur est ensuite fermé et il est mis sous pression et porté à la température désirée pendant le temps nécessaire pour que le CO₂ se trouve sous forme supercritique et pour obtenir l'imprégnation de l'excipient pharmaceutique polymérique par la substance active, sans liquéfaction ou ramollissement de cet excipient. En effet durant toute cette étape b), l'excipient pharmaceutique polymérique reste sous forme solide et en particulier sous forme de poudre. Il n'y a aucune modification visible à l'oeil nu de la structure de l'excipient pharmaceutique polymérique. Particulièrement, si la substance active est elle-même sous forme solide et en particulier sous forme de poudre, la substance active reste également sous forme solide et en particulier sous forme de poudre pendant toute la durée de l'étape b) et il n'y a pas de modification visible à l'oeil nu de la structure de la substance active.

Dans un mode de réalisation particulier, la température de l'étape b) est comprise entre 40 et 85°C de façon par exemple entre 50 et 80°C.

Dans un autre mode de réalisation particulier, la pression de l'étape b) est comprise entre 100 et 160 bars, par exemple égale à 150 bars.

Dans encore un autre mode de réalisation particulier, il n'y a pas d'étape de lavage par CO₂ supercritique après l'étape b) et/ou d'étape de séchage et/ou d'étape de pulvérisation, en particulier à l'aide d'une buse.

Dans un mode de réalisation particulier, le temps de contact de l'étape b) est compris entre 1 et 3 heures, par exemple égal à deux heures.

L'étape b) du procédé selon la présente invention est mise en oeuvre sans agitation, ce qui rend le procédé facilement industrialisable. En effet au vu des excipients pharmaceutiques utilisés dans le cadre de la présente invention, et en particulier en raison du fait que l'excipient conserve sa forme solide pendant toute la durée de l'étape b), l'utilisation de l'agitation dans un réacteur haute pression n'est possible qu'au stade du laboratoire (utilisation d'un réacteur de quelques litres) et non au stade industriel. En effet, pour transmettre le mouvement du moteur (qui est à la pression atmosphérique) à un arbre d'agitation (qui est sous haute pression) on ne peut pas assurer l'étanchéité avec des joints "classique". On utilise donc un entrainement magnétique: un aimant qui entraine un autre aimant, cela limite les possibilités en termes de couple d'agitation.

Dans le cadre de la présente invention, le procédé est mis en oeuvre en l'absence de solvant additionnel. L'eau est en particulier absente de l'étape b) selon la présente invention. Le mélange obtenu à l'étape a) est sec. De même, le produit obtenu à la fin de l'étape b) est sec.

Ainsi, le seul solvant présent lors de l'étape b) de la présente invention est le CO₂ à l'état supercritique.

Avantageusement, le procédé selon la présente invention est mis en oeuvre en absence de tout autre fluide, par exemple, liquide, par exemple l'eau, en dehors du CO₂ supercritique.

Ainsi, dans un mode de réalisation particulier de l'invention, les seuls ingrédients en présence sont : la substance active, l'excipient pharmaceutique et le CO₂ supercritique, en particulier lors de l'étape b).

L'étape c) permet de récupérer et d'isoler l'excipient pharmaceutique polymérique imprégné obtenu suite à l'étape b) et éventuellement de le séparer de la substance active qui n'a pas imprégné l'excipient polymérique et/ou de l'excipient pharmaceutique polymérique qui n'a pas été imprégné. Pour cela, le réacteur utilisé à l'étape b) est dépressurisé et refroidit. Le CO₂ est éliminé sous forme de gaz.

L'excipient pharmaceutique polymérique imprégné se trouve sous forme solide.

Si l'excipient pharmaceutique polymérique était sous forme de poudre au début de l'étape b), alors il reste sous forme de poudre après son imprégnation, c'est-à-dire à la fin de l'étape b) et lors de l'étape c). En particulier, en raison de l'absence d'eau lors de l'étape b) du procédé selon la présente invention la poudre obtenue est une poudre sèche. Il n'est donc pas nécessaire de sécher cette poudre.

La substance active qui imprègne l'excipient pharmaceutique polymérique est sous forme amorphe stabilisée. Ainsi cette substance active conserve cette forme amorphe durant toute sa durée de conservation et en particulier au moins 2 mois, de en particulier jusqu'à un an. Par le terme « substance active sous forme amorphe stabilisée », on entend au sens de la présente invention, toute substance active ayant une propension à cristalliser ou recristalliser maintenue sous forme amorphe sur une durée plus longue que si elle n'est pas traitée par le procédé selon la présente invention, en particulier sur une durée d'au moins 6 mois, encore plus particulièrement sur une durée d'au moins 1 an.

L'excipient pharmaceutique imprégné par la substance active peut être utilisé dans la préparation de toute composition pharmaceutique, cosmétique ou nutraceutique dans laquelle la présence de la substance active sous forme amorphe est nécessaire.

En particulier il peut être utilisé dans la fabrication de pilules, comprimés ou gélules destinées à la voie orale.

Le procédé selon la présente invention est particulièrement intéressant pour potentialiser la fonctionnalité de l'excipient pharmaceutique. Ainsi, il est possible en utilisant un excipient pharmaceutique approprié d'obtenir un principe actif avec un relargage différé pour un principe actif très soluble dans l'eau ou de rendre soluble un principe actif peu soluble dans l'eau.

La présente invention concerne en outre un excipient pharmaceutique polymérique sous forme solide non poreux imprégné par une substance active caractérisé en ce qu'il est susceptible d'être obtenu par le procédé selon la présente invention tel que décrit ci-dessus, en ce que la substance active est sous forme amorphe et est soluble dans l'eau et en ce que l'excipient pharmaceutique polymérique n'est pas de la polyvinylpyrrolidone non réticulé et est insoluble dans le CO₂ supercritique.

Dans un mode de réalisation particulier de l'invention, la substance active est du milnacipran, en particulier sous forme de chlorhydrate, par exemple l'énantiomère (1S, 2R).

Dans un autre mode de réalisation particulier, l'excipient pharmaceutique polymérique est un agent de charge, en particulier choisi parmi la méthylcellulose, l'hydroxypropylméthylcellulose et leurs mélanges.

La présente invention concerne en outre l'excipient pharmaceutique polymérique sous forme solide imprégné par du milnacipran selon la présente invention pour utilisation à titre de médicament.

Elle concerne enfin l'excipient pharmaceutique polymérique sous forme solide imprégné par du milnacipran, en particulier sous forme de l'énantiomère (1S, 2R), selon la présente invention pour utilisation à titre de médicament ayant une action antidépresseur et/ou destiné au traitement de la dépression, telle que la dépression profonde, la dépression résistante, la dépression psychotique, la dépression induite par des traitements avec l'interféron, les états dépressifs, le syndrome maniaco-dépressif, les états de dépression saisonniers, les épisodes dépressifs en relation avec l'état de santé général et la dépression dues à des substances altérants l'humeur, des maladies bipolaires, de la schizophrénie, de l'anxiété générale, des états moroses et de marasmes, des maladies dues au stress, des attaques de panique, des phobies, des troubles post-traumatiques, de la phobie sociale, des troubles obsessionnels- compulsifs, des désordres du comportement, de la désintoxication toxicomaniaque, de la dépression du système immunitaire, des syndromes de fatigue et de douleur associée, du syndrome de fatigue chronique, de l'autisme, des troubles de l'attention dues à l'hyperactivité, des troubles du sommeil, des troubles dysphoriques prémenstruels, des maladies cardiovasculaires, des maladies neurodégénératives et des syndromes d'anxiété et de dépression associés (maladie d'Alzheimer, chorée de Huntington, maladie de Parkinson) incontinence urinaire des troubles de l'alimentation, de la boulimie névrotique, de l'anorexie névrotique, de l'obésité, de l'apathie, de la migraine, et/ou du syndrome du colon irritable et/ou destiné au traitement du syndrome fibromyalgique et/ou d'autres désordres fonctionnels, et/ ou au traitement de troubles psychiatriques, en particulier des désordres du système nerveux central, plus particulièrement tout en réduisant le risque de tendances suicidaires.

Les exemples suivants sont donnés à titre indicatif non limitatif

### EXEMPLES

### EXEMPLE 1 : avec le kétoprofène

### 1a Kétoprofène / PVP (polyvinylpyrrolidone) réticulé

Mélange de poudre:1g de Kétoprofène sous forme cristalline (SIGMA) + 2g de Polyplasdone XL 10(ISP) manuellement au mortier (étape a) du procédé selon la présente invention).

Traitement avec CO₂ supercritique (SC) à 150 bars, 80 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### 1b Kétoprofène / méthylcellulose

Mélange de poudre:1g de Kétoprofène sous forme cristalline(SIGMA) + 2g de Metolose SM 4 (SEPPIC) manuellement au mortier (étape a) du procédé selon la présente invention)

Traitement avec CO₂ SC à 150 bars, 80 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### 1c Kétoprofène / hydroxypropylméthylcellulose (HPMC)

Mélange de poudre:1g de Kétoprofène sous forme cristalline (SIGMA) + 2g de Benecel MP 843 HPMC (ASHLAND) manuellement au mortier (étape a) du procédé selon la présente invention)

Traitement avec CO₂ SC à 150 bars, 80 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### 1d (exemple comparatif) Kétoprofène / Lactose

Mélange de poudre:1g de Kétoprofène sous forme cristalline (SIGMA) + 2g de lactose manuellement au mortier (étape a) du procédé selon la présente invention)

Traitement avec CO₂ SC à 150 bars, 80 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### Protocole analytique et résultats concernant l'amorphisation de la substance active (kétoprofène):

L'analyse calorimétrique différentielle ou DSC permet d'observer les accidents thermiques caractéristiques d'un corps (déshydratation, cristallisation, fusion,...).

Ces analyses ont été effectuées sur un appareil DSC, METTLER-TOLEDO. On place une quantité connue de poudre dans une capsule en aluminium dans le creuset « échantillon » de l'appareil. On effectue une rampe de température de 20 à 110 °C à une vitesse de 5 °C/min.

La température de fusion du kétoprofène est de 94°C, son enthalpie de fusion est égale à 116 J/g. On mesure l'amorphisation en comparant le pic de fusion de la substance active dans la poudre après traitement avec « CO₂ supercritique » selon la présente invention à celui du mélange physique correspondant.

Si le pic de fusion du kétoprofène disparaît complètement alors l'amorphisation est considérée égale à 100%.

Le tableau 1 ci après montre le % d'amorphisation de la substance active après imprégnation dans l'excipient pharmaceutique polymérique selon l'invention (exemple 1a, 1b et 1c) ou dans le lactose (exemple 1d) à T0 (juste après le procédé selon l'invention) ou après stockage pendant 2 mois, 4 mois, 7 mois ou 1 an.

**Tableau 1**

| **Amorphisation** | **T0** | **T0 + 2 mois** | **T0 + 4 mois** | **T0 + 7 mois** | **T0** + **1 an** |
|---|---|---|---|---|---|
| Exemple 1a | 100 % | 100 % | 100 % | 100 % | 100 % |
| Exemple 1b B | 100 % | 100 % | 100 % | 100 % | |
| Exemple 1c | 100 % | 100 % | 100 % | 100 % | |
| Exemple 1d | 0 % | | | | |

Les échantillons ont été stockés sans aucune précaution particulière. L'analyse DSC a été réalisée plusieurs fois durant une année et montre que l'amorphisation de la substance active est stabilisée dans le cas des exemples 1a, 1b et 1c.

Après analyse DSC, on constate que le taux d'amorphisation du kétoprofène est nul dans le cas de l'exemple 1d.

L'utilisation de lactose comme excipient ne conduit pas à l'obtention d'une dispersion solide stabilisée. De même, pour tous les essais réalisés avec une cyclodextrine dans les différents exemples du brevet WO2004/096284: l'absence d'eau ajoutée lors de l'étape b) du procédé selon la présente invention empêche la complexation, il n'y a donc pas d'amorphisation.

Le lactose (deux unités glucose) et la cyclodextrine (sept unités glucose) sont des oligosaccharides et non pas des polymères.

### Accélération de la cinétique de dissolution avec les exemples 1a, 1b et 1c (super-désintégrant)

### Protocole analytique :

Dans un erlenmeyer de 100 ml, introduire une prise d'essai équivalente à 50 mg exactement mesuré de kétoprofène pur ou imprégnant le PVP réticulé. Ajouter 50 ml d'eau. Mettre sous agitation magnétique à 400 tour par minutes dans un bain-marie à 37°C +/- 2°C. Effectuer un prélèvement de 2 ml sous agitation magnétique à 5, 15 et 30 minutes. Filtrer ces prélèvements sur filtres polypropylène 0,45 µm Gelman GHP Acrodisc. La solution doit être limpide.

Déterminer la teneur en kétoprofène par chromatographie liquide.

Les résultats sont rassemblés dans le tableau 2 suivant:

**Tableau 2**

| | EXCIPIENT | Dosage % kétoprofène | Dissolution (µg/ml) | | |
|---|---|---|---|---|---|
| | | | 15 min | 30 min | 60 min |
| kétoprofène | / | / | 173 | 179 | 171 |
| Mélange Physique | PVP réticulé XL10 | 29,3 | 157 | 177 | 172 |
| Exemple 1a | PVP réticulé XL10 | 29,3 | 279 | 310 | 311 |
| Exemple 1b | Metolose SM 4 | 31,21 | 399 | 437 | 459 |
| Exemple 1c | Benecel HPMC | 31,65 | 399 | 495 | 556 |
| Exemple 1d | Lactose | 30,13 | 104 | 176 | 183 |

Le procédé selon la présente invention permet d'améliorer la cinétique de dissolution du kétoprofène.

### EXEMPLE 2 : avec la vinflunine

### 2a :Vinflunine base / carboxyméthylcellulose

Mélange de poudre:1g de Vinflunine base sous forme cristalline + 2g de Croscarmellose de Sodium manuellement au mortier (étape a) du procédé selon la présente invention)

Traitement avec CO₂ SC à 150 bars, 50 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### Accélération de la cinétique de dissolution avec l'exemple 2a (super-désintégrant).

### Protocole analytique :

Dans un erlenmeyer de 100 ml, introduire une prise d'essai équivalente à 50 mg exactement mesuré de vinflunine. Ajouter 50 ml de tampon pH 6,8. Mettre sous agitation magnétique à 400 rpm. Effectuer un prélèvement de 2 ml sous agitation magnétique à 15, 30, 60 et 120 minutes. Filtrer ces prélèvements sur filtres polypropylène 0.45 µm Gelman GHP Acrodisc. La solution doit être limpide.

Déterminer la teneur en vinflunine par chromatographie liquide.

Les résultats sont rassemblés dans le tableau 3 suivant:

**Tableau 3**

| | EXCIPIENT | Dosage % vinflunine | Dissolution (µg/ml) | | | |
|---|---|---|---|---|---|---|
| | | | 15 min | 30 min | 60 min | 120 min |
| Vinflunine base | / | / | 252 | 387 | 520 | 606 |
| Mélange Physique | Croscarmellose de Sodium | 28,9 | 274 | 372 | 416 | 636 |
| Exemple 2a | Croscarmellose de Sodium | 28,9 | 765 | 801 | 854 | 807 |

Le procédé selon la présente invention permet d'améliorer la cinétique de dissolution de la vinflunine base.

### Augmentation de la biodisponibilité avec l'exemple 2a

### Protocole analytique :

L'étude a été menée en Pk exploratoire chez le rat: 2 mg/kg en dose unique, voie orale
Véhicule d'administration : eau distillée (Aguettant)
Animaux mis à jeun (fasted rats)
Méthode bioanalyse LC/MS/MS (chromatographie liquide/spectrométrie de masse/spectrométrie de masse) en détection Electrospray mode positif (ESI+)
Analyse Pharmacocinétique par Kinetica (Thermo Instruments, US)

Les résultats sont rassemblés dans le tableau 4 suivant:

**Tableau 4**

| Temps (min) | Vinflunine base | Exemple 2a |
|---|---|---|
| 0 | 0 | 0 |
| 0,25 | 19,37 | 39,13 |
| 0,5 | 11,75 | 32,30 |
| 1 | 17,90 | 28,07 |
| 2 | 14,93 | 27,23 |
| 4 | 13,80 | 28,70 |
| 6 | 12,03 | 19,93 |
| 8 | 10,88 | 18,27 |
| 24 | 2,59 | 3,39 |

| Paramètres pharmacocinétiques | | |
|---|---|---|
| Cmax (ng/ml) | 19,37 | 39,13 |
| Tmax (h) | 0,25 | 0,25 |
| AUClast (ng.h/ml) | 199,80 | 340,10 |
| ½ vie (h) | 7,90 | 6,90 |
| CL (L/h) | 8,70 | 5,40 |

Le procédé selon la présente invention permet d'améliorer la bio-disponibilité orale de la vinflunine base (augmentation de l'AUC et du C max).

### EXEMPLE 3 : avec le milnacipran

### 3a Milnacipran / méthylcellulose

Mélange de poudre:1,5g de chlorhydrate de Milnacipran (PFM) sous forme cristalline + 7,5g de Metolose SM4 (Seppic) manuellement au mortier (étape a) du procédé selon la présente invention)

Traitement avec CO₂ SC à 150 bars, 80 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### 3b Milnacipran / HPMC

Mélange de poudre:1,5g de chlorhydrate de Milnacipran (PFM) sous forme cristalline + 7,5g d'HPMC (Benecel) manuellement au mortier (étape a) du procédé selon la présente invention)

Traitement avec CO₂ SC à 150 bars, 80 °C, 2h (étape b) du procédé selon la présente invention) par mise sous pression d'un autoclave haute pression à température régulée contenant le mélange.

Récupération de la poudre (étape c) du procédé selon la présente invention) par dépressurisation de l'autoclave.

### Effet retard - relargage différé (Controlled Release System), avec exemple 3a et 3b Protocole analytique :

Dans un erlenmeyer de 100 ml, introduire une prise d'essai équivalente à 100 mg exactement mesuré de milnacipran. Ajouter 50 ml d'eau. Mettre sous agitation magnétique à 400 rpm. Effectuer un prélèvement de 2 ml sous agitation magnétique à 1, 5, 15, 30 et 60 minutes. Filtrer ces prélèvements sur filtres polypropylène 0.45 µm Gelman GHP Acrodisc. La solution doit être limpide. Effectuer une dilution au 1/5 dans de la phase mobile.
Déterminer la teneur en milnacipran par chromatographie liquide.
Les résultats sont rassemblés dans le tableau 5 suivant.

**Tableau 5 :**

| | EXCIPIENT | Dosage % milnacipran | Dissolution (g/l) | | | | |
|---|---|---|---|---|---|---|---|
| | | | 1 min | 5 min | 15 min | 30 min | 60 min |
| Chlorhydrate de Milnacipran | / | / | 10 | 10,1 | 10,1 | 10,1 | 10,2 |
| Exemple 3a | méthylcellulose | 16,8 | 3,2 | 4,0 | 5,4 | 6,7 | 8,2 |
| Exemple 3b | HPMC | 14,9 | 0,3 | 0,6 | 0,9 | 1,2 | 1,7 |

Le procédé selon l'invention permet le relargage différé du Chlorhydrate de Milnacipran (un principe actif complètement soluble).

### Exemple comparatif 4

Cet exemple a été mis en oeuvre en suivant le protocole de la demande de brevet WO 99/25322. Dans ce document, l'étape d'extraction pour obtenir le «CO₂ saturé» est l'étape limitante, car les principes actifs sont souvent considérés comme insoluble dans le CO₂ supercritique.

Extraction /percolation Milnacipran / hydroxypropylmethylcellulose (HPMC) 40 grammes de Milnacipran sont placés dans une cellule d'extraction, 8 grammes de Benecel MP 843 (hydroxypropylmethylcellulose) sont placés dans une colonne en aval de la cellule. Un flux de 5 kg/h de CO₂ SC à 150 bars 70 °C est appliqué pendant 3 heures.

La poudre récupérée dans la colonne est analysée par HPLC : aucune trace de Milnacipran n'est détectée. L'actif, insoluble, n'a pas été extrait et n'a donc pas percolé à travers l'HPMC.

## Revendications

1. Procédé batch d'imprégnation d'un excipient pharmaceutique polymérique non poreux par une substance active, **caractérisé en ce qu'**il comprend les étapes successives suivantes :
a) mélange de la substance active et de l'excipient pharmaceutique polymérique non poreux, l'excipient pharmaceutique polymérique étant sous forme solide et insoluble dans le CO₂ supercritique et n'étant pas de la polyvinylpyrrolidone non réticulé;
b) mise en oeuvre d'une étape de diffusion moléculaire en l'absence d'eau par mise en contact en mode statique sans agitation du mélange obtenu à l'étape a) avec du CO₂ supercritique à une pression comprise entre 80 et 170 bars, à une température comprise entre 31 et 90°C pendant entre 1 et 6 heures,
c) récupération de l'excipient pharmaceutique polymérique imprégné par la substance active obtenu à l'étape b), l'excipient pharmaceutique polymérique imprégné n'étant pas poreux et étant sous forme solide et la substance active étant sous forme amorphe
et en ce que le procédé est mis en oeuvre en l'absence de solvant additionnel.

2. Procédé selon la revendication 1, **caractérisé en ce que** l'excipient pharmaceutique polymérique est choisi parmi les agents désintégrant et les agents de charge, en particulier dans le groupe constitué par la polyvinylpyrrolidone réticulé, la carboxyméthylcellulose par exemple de sodium, l'hydroxypropylméthylcellulose, la méthylcellulose et leurs mélanges.

3. Procédé selon l'une quelconque des revendications précédentes, **caractérisé en ce que** le ratio massique entre le principe actif et l'excipient pharmaceutique polymérique est compris entre 1 et 60%, par exemple compris entre 10 et 35%.

4. Procédé selon l'une quelconque des revendications précédentes, **caractérisé en ce que** la substance active est choisie dans le groupe constitué par le kétoprofène, la vinflunine, le milnacipran, le fénofibrate, le sulfate ferreux monohydrate et le sulfate ferreux heptahydrate.

5. Procédé selon l'une quelconque des revendications précédentes, **caractérisé en ce que** la température de l'étape b) est comprise entre 40 et 85°C par exemple entre 50 et 80°C.

6. Procédé selon l'une quelconque des revendications précédentes, **caractérisé en ce que** la pression de l'étape b) est comprise entre 100 et 160 bars, par exemple égale à 150 bars.

7. Procédé selon l'une quelconque des revendications précédentes, **caractérisé en ce que** le temps de contact de l'étape b) est compris entre 1 et 3 heures, par exemple égal à deux heures.

8. Procédé selon l'une quelconque des revendications précédentes, **caractérisé en ce que** le principe actif lors de l'étape a) est sous forme de poudre, par exemple sous forme cristalline.

9. Excipient pharmaceutique polymérique sous forme solide non poreux imprégné par une substance active **caractérisé en ce qu'**il est susceptible d'être obtenu par le procédé selon l'une quelconque des revendications précédentes, **en ce que** la substance active est sous forme amorphe et est soluble dans l'eau, **en ce que** l'excipient pharmaceutique polymérique n'est pas de la polyvinylpyrrolidone non réticulé et est insoluble dans le CO₂ supercritique, et **en ce que** la substance active est du milnacipran, par exemple sous forme de chlorhydrate.

10. Excipient pharmaceutique polymérique sous forme solide imprégné par une substance active selon la revendication 9, **caractérisé en ce que** l'excipient est un agent de charge, par exemple choisi parmi la méthylcellulose, l'hydroxypropylméthylcellulose et leurs mélanges.

11. Excipient pharmaceutique polymérique sous forme solide imprégné par du milnacipran selon l'une quelconque des revendications 9 ou 10 pour utilisation à titre de médicament.

12. Excipient pharmaceutique polymérique sous forme solide imprégné par du milnacipran, en particulier sous forme de l'énantiomère (1S, 2R), selon la revendication 11 pour utilisation à titre de médicament ayant une action antidépresseur et/ou destiné au traitement de la dépression, telle que la dépression profonde, la dépression résistante, la dépression psychotique, la dépression induite par des traitements avec l'interféron, les états dépressifs, le syndrome maniaco-dépressif, les états de dépression saisonniers, les épisodes dépressifs en relation avec l'état de santé général et la dépression dues à des substances altérants l'humeur, des maladies bipolaires, de la schizophrénie, de l'anxiété générale, des états moroses et de marasmes, des maladies dues au stress, des attaques de panique, des phobies, des troubles post-traumatiques, de la phobie sociale, des troubles obsessionnels- compulsifs, des désordres du comportement, de la désintoxication toxicomaniaque, de la dépression du système immunitaire, des syndromes de fatigue et de douleur associée, du syndrome de fatigue chronique, de l'autisme, des troubles de l'attention dues à l'hyperactivité, des troubles du sommeil, des troubles dysphoriques prémenstruels, des maladies cardiovasculaires, des maladies neurodégénératives et des syndromes d'anxiété et de dépression associés (maladie d'Alzheimer, chorée de Huntington, maladie de Parkinson) incontinence urinaire des troubles de l'alimentation, de la boulimie névrotique, de l'anorexie névrotique, de l'obésité, de l'apathie, de la migraine, et/ou du syndrome du colon irritable et/ou destiné au traitement du syndrome fibromyalgique et/ou d'autres désordres fonctionnels, et/ ou au traitement de troubles psychiatriques, en particulier des désordres du système nerveux central, plus particulièrement tout en réduisant le risque de tendances suicidaires.

## Patentansprüche

1. Batch-Verfahren zum Tränken eines nicht porösen polymeren pharmazeutischen Trägers mit einem Wirkstoff, **dadurch gekennzeichnet, dass** es die folgenden Schritte umfasst:
a) Mischen des Wirkstoffs und des nicht porösen polymeren pharmazeutischen Trägers, wobei der polymere pharmazeutische Träger in fester Form vorliegt und in superkritischem CO₂ unlöslich ist und kein unvernetztes Polyvinylpyrrolidon ist;
b) Durchführen eines Schrittes der molekularen Diffusion in Abwesenheit von Wasser durch Inkontaktbringen auf statische Weise ohne Schütteln des in Schritt a) erhaltenen Gemischs mit superkritischem CO₂ bei einem Druck zwischen 80 und 170 bar und bei einer Temperatur zwischen 31 und 90 °C über einen Zeitraum zwischen 1 und 6 Stunden,
c) Auffangen des in Schritt b) erhaltenen, mit dem Wirkstoff getränkten polymeren pharmazeutischen Trägers, wobei der getränkte polymere pharmazeutische Träger nicht porös ist und in fester Form vorliegt und wobei der Wirkstoff in amorpher Form vorliegt,
und dass das Verfahren in Abwesenheit eines zusätzliches Lösemittels durchgeführt wird.

2. Verfahren nach Anspruch 1, **dadurch gekennzeichnet, dass** der polymere pharmazeutische Träger ausgewählt aus Sprengmitteln und Füllstoffen, insbesondere aus der Gruppe bestehend aus vernetztem Polyvinylpyrrolidon, Carboxymethylcellulose, zum Beispiel deren Natriumsalz, Hydroxypropylmethylcellulose, Methylcellulose und deren Gemischen.

3. Verfahren nach einem der vorhergehenden Ansprüche, **dadurch gekennzeichnet, dass** das Masseverhältnis zwischen dem Wirkstoff und dem polymeren pharmazeutischen Träger zwischen 1 und 60 %, zum Beispiel zwischen 10 und 35 % beträgt.

4. Verfahren nach einem der vorhergehenden Ansprüche, **dadurch gekennzeichnet, dass** der Wirkstoff ausgewählt ist aus der Gruppe bestehend aus Ketoprofen, Vinflunin, Milnacipran, Fenofibrat, Eisen(II)sulfat-Monohydrat und Eisen(II)sulfat-Heptahydrat.

5. Verfahren nach einem der vorhergehenden Ansprüche, **dadurch gekennzeichnet, dass** die Temperatur in Schritt b) zwischen 40 und 85 °C, zum Beispiel zwischen 50 und 80 °C beträgt.

6. Verfahren nach einem der vorhergehenden Ansprüche, **dadurch gekennzeichnet, dass** der Druck in Schritt b) zwischen 100 und 160 bar beträgt, zum Beispiel gleich 150 bar ist.

7. Verfahren nach einem der vorhergehenden Ansprüche, **dadurch gekennzeichnet, dass** die Kontaktzeit in Schritt b) zwischen 1 und 3 Stunden, zum Beispiel gleich zwei Stunden ist.

8. Verfahren nach einem der vorhergehenden Ansprüche, **dadurch gekennzeichnet, dass** der Wirkstoff in Schritt a) in Form von Pulver, zum Beispiel in kristalliner Form vorliegt.

9. Mit einem Wirkstoff getränkter, nicht poröser polymerer pharmazeutischer Träger in fester Form, **dadurch gekennzeichnet, dass** er durch das Verfahren nach einem der vorhergehenden Ansprüche erhalten werden kann, dass der Wirkstoff in amorpher Form vorliegt und in Wasser löslich ist, dass der polymere pharmazeutische Träger kein unvernetztes Polyvinylpyrrolidon ist und in superkritischem CO₂ unlöslich ist, und dass der Wirkstoff Milnacipran, zum Beispiel in Form von Chlorhydrat ist.

10. Mit einem Wirkstoff getränkter polymerer pharmazeutischer Träger in fester Form nach Anspruch 9, **dadurch gekennzeichnet, dass** der Träger ein Füllstoff ist, der zum Beispiel ausgewählt ist aus Methylcellulose, Hydroxypropylmethylcellulose und deren Gemischen.

11. Mit Milnacipran getränkter polymerer pharmazeutischer Träger in fester Form nach einem der Ansprüche 9 oder 10 zur Verwendung als Arzneimittel.

12. Mit Milnacipran getränkter polymerer pharmazeutischer Träger in fester Form, insbesondere in enantiomerer Form (1S, 2R), nach Anspruch 11 zur Verwendung als Arzneimittel mit einer antidepressiven Wirkung und/oder zur Behandlung von Depressionen, wie tiefe Depression, resistente Depression, psychotische Depression, durch Behandlungen mit Interferon hervorgerufene Depression, depressive Zustände, manisch-depressives Syndrom, Zustände saisonal abhängiger Depression, depressive Episoden in Verbindung mit dem allgemeinen Gesundheitszustand und durch stimmungsbeeinträchtigende Substanzen bedingte Depression, bipolare Erkrankungen, Schizophrenie, allgemeine Angststörung, moröse Zustände und Marasmen, stressbedingte Erkrankungen, Panikattacken, Phobien, posttraumatische Störungen, soziale Phobie, Zwangsstörungen, Verhaltensstörungen, Drogenentzug, Depression des Immunsystems, Syndrome der Erschöpfung und damit verbundener Schmerzen, chronisches Erschöpfungssyndrom, Autismus, hyperaktivitätsbedingte Aufmerksamkeitsstörungen, Schlafstörungen, prämenstruelle dysphorische Störungen, kardiovaskuläre Erkrankungen, neurodegenerative Erkrankungen und damit verbundene Angst- und Depressionssyndrome (Alzheimer-Krankheit, Huntingtonsche Chorea, Parkinson-Krankheit), Urininkontinenz, Ernährungsstörungen, neurotische Bulimie, neurotische Anorexie, Fettleibigkeit, Apathie, Migräne und/oder Reizdarmsyndrom, und/oder für die Behandlung des Fibromyalgiesyndroms und/oder anderen funktionellen Störungen, und/oder zur Behandlung von psychiatrischen Störungen, insbesondere von Störungen des zentralen Nervensystems, weiter insbesondere unter gleichzeitiger Reduzierung der Gefahr von suizidalen Tendenzen.

## Claims

1. Batch method for impregnating a polymeric pharmaceutical carrier with an active substance, **characterised in that** it comprises the following consecutive steps:
a) mixing the active substance and the non-porous polymeric pharmaceutical carrier, the pharmaceutical carrier being in solid form and insoluble in supercritical CO₂ and not being non-cross-linked polyvinylpyrrolidone;
b) performing a molecular diffusion step in the absence of water by contacting, in static mode without stirring, the mixture obtained in step a) with supercritical CO₂ at a pressure between 80 and 170 bar and at a temperature between 31 and 90°C for between 1 and 6 hours,
c) recovering the polymeric pharmaceutical carrier impregnated with the active substance obtained in step b), the impregnated pharmaceutical carrier being non-porous and being in solid form and the active substance being in amorphous form,
and in that the method is implemented in the absence of an additional solvent.

2. Method according to claim 1, **characterised in that** the polymeric pharmaceutical carrier is selected from disintegrating agents and filler agents, in particular in the group consisting of cross-linked polyvinylpyrrolidone, carboxymethyl cellulose, of sodium for example, hydroxypropyl methyl cellulose, methyl cellulose and the mixtures thereof.

3. Method according to any of the preceding claims, **characterised in that** the mass ratio between the active substance and the polymeric pharmaceutical carrier is between 1 and 60%, for example between 10 and 35%.

4. Method according to any of the preceding claims, **characterised in that** the active substance is selected in the group consisting of ketoprofen, vinflunine, milnacipran, fenofibrate, iron sulphate monohydrate and iron sulphate heptahydrate.

5. Method according to any of the preceding claims, **characterised in that** the temperature in step b) is between 40 and 85°C, for example between 50 and 80°C.

6. Method according to any of the preceding claims, **characterised in that** the pressure in step b) is between 100 and 160 bar, for example equal to 150 bar.

7. Method according to any of the preceding claims, **characterised in that** the contact time in step b) is between 1 and 3 hours, for example equal to two hours.

8. Method according to any of the preceding claims, **characterised in that** the active substance during step a) is in powder form, for example in crystalline form.

9. Polymeric pharmaceutical carrier in non-porous solid form impregnated with an active substance **characterised in that** it is obtainable with the method according to any of the preceding claims, **in that** the active substance is in amorphous form and is soluble in water, **in that** the polymeric pharmaceutical carrier is not non-cross-linked polyvinylpyrrolidone and is insoluble in supercritical CO₂, and **in that** the active substance is milnacipran, for example in hydrochloride form.

10. Polymeric pharmaceutical carrier in solid form impregnated with an active substance according to claim 9, **characterised in that** the carrier is a filler agent, for example selected from methyl cellulose, hydroxypropyl methyl cellulose and the mixtures thereof.

11. Polymeric pharmaceutical carrier in solid form impregnated with milnacipran according to any of claims 9 or 10 for use as a medicinal product.

12. Polymeric pharmaceutical carrier in solid form impregnated with milnacipran, in particular in the (1S, 2R) enantiomer form, according to claim 11 for use as a medicinal product having an antidepressant action and/or intended for treating depression, such as deep depression, resistant depression, psychotic depression, depression induced by interferon treatments, states of depression, manic depression syndrome, seasonal states of depression, episodes of depression linked with general health and depression due to mood-altering substances, bipolar disorders, schizophrenia, general anxiety, morosis and marasmus, stress-related disorders, panic attacks, phobias, post-traumatic disorders, social phobia, obsessive-compulsive disorders, behavioural disorders, drug addiction detoxification, immune system depression, fatigue and associated pain syndromes, chronic fatigue syndrome, autism, hyperactive attention disorders, sleep disorders, premenstrual dysphoric disorders, cardiovascular diseases, neurodegenerative diseases and anxiety and associated depression syndromes (Alzheimer's disease, Huntington's chorea, Parkinson's disease), urinary incontinence, eating disorders, bulimia nervosa, anorexia nervosa, obesity, apathy, migraine and/or irritable bowel syndrome and/or intended for treating fibromyalgia syndrome and/or other functional disorders, and/or for treating psychiatric disorders, particularly central nervous system disorders, more specifically while reducing the risk of suicidal tendencies.
